# EUROPÄISCHE PATENTSCHRIFT

(11) **EP 1 279 387 B1**
(45) Veröffentlichungstag und Bekanntmachung des Hinweises auf die Patenterteilung: **27.02.2008**
(21) Anmeldenummer: 02013772.5
(22) Anmeldetag: 21.06.2002
(51) Int. Cl.: A61F 9/02

(54) **Sichtscheibe für eine Skibrille und Verfahren zu deren Herstellung**
Vision glass for ski glasses and method for manufacture
Verre pour lunettes de ski et procédé de sa fabrication

(30) Priorität: 27.07.2001 DE 10136806
(43) Veröffentlichungstag der Anmeldung: 29.01.2003
(73) Patentinhaber: Uvex Sports GmbH & Co. KG, 90763 Fürth (DE)
(72) Erfinder: Grau, Werner, 86316 Haberskirch (DE); Huber, Franz, 93462 Lam (DE)
(74) Vertreter: Schneck, Herbert

(56) Entgegenhaltungen:
- WO-A-99/44555
- DE-A- 3 935 994
- DE-U- 8 628 009
- US-A- 3 841 740
- US-A- 5 167 756
- US-A- 5 985 069

## Beschreibung

Die Erfindung richtet sich auf eine Sichtscheibe für eine Skibrille umfassend eine äußere und eine innere Scheibe, wobei die äußere Scheibe aus Cellulosepropionat, Celluloseacetat oder Polycarbonat und die innere Scheibe aus Cellulosepropionat oder Celluloseacetat besteht, welche über ein längs des Randbereiches umlaufendes, elastisches Abstandshalte-Dichtungs-Element miteinander verklebt sind. Eine gattungsgemäße Sichtscheibe ist aus der WO 99/44555 A bekannt.

Derartige sogenannte Doppelscheiben sind von Skibrillen und MotorradHelm-Visieren seit langem bekannt. Sie ermöglichen es, für die inneren und die äußeren Scheiben unterschiedliche Materialien zu verwenden, also für die äußere Scheibe besonders kratzfestes Material und für die innere Scheibe Material, das sich gut mit einer Anti-Beschlag-Beschichtung versehen lässt. Weiterhin wird durch das eingeschlossene Luftpolster ebenfalls einem Beschlagen der Scheibe entgegengewirkt.

Zur Herstellung solcher Doppelscheiben-Anordnungen sind unterschiedliche Techniken oder Spritzgießen bekannt. Beispielsweise wird die äußere Scheibe durch Tiefziehen unter Ausbildung eines gegen die eigentliche Scheibenebene vorstehenden Außenrandes hergestellt und die innere Scheibe dann an diesem Rand durch Verkleben oder Ultraschallverschweißen befestigt. Das Tiefziehen oder Gießen der äußeren Scheibe ist kostenaufwendig und der entstehende Verbund ist außerordentlich starr. Demgegenüber müssen Skibrillen-Scheiben zur Vermeidung von Verletzungen bei Stürzen bzw. um ein Zerbrechen zu verhindern möglichst elastisch und flexibel sein.

Weiterhin ist es bekannt, das Material des Abstandshalte-Dichtungs-Elements aus flächigem Material, insbesondere Schaumstoffmaterial, zu stanzen und die Scheiben dann hiermit mittels Klebstoff zu verkleben. Durch dieses Stanzen entsteht eine erhebliche Menge von Abfall und das Verkleben ist sehr arbeitsaufwendig.

Aus DE 295 16 680 U1 ist es bekannt, die beiden Komponenten eines PUR-Schaums numerisch gesteuert längs des Außenrandes einer Scheibe für eine Skibrille aufzubringen, um auf diese Weise einen Schaumstoffstreifen zu erzeugen, der am Gesicht des Trägers anliegt.

Aus JP-Patent Abstracts 11 079 797 A und 09 086 979 A ist es bekannt, zwischen zwei planparallelen Glasscheiben eine Dichtung aus Hydrocarbonharz oder aus teilweise vulkanisiertem Butylgummi anzubringen

US-A-5,985,069 beschreibt eine Doppelscheibe für Lichtschutzzwecke, wobei zwischen die Scheiben eine Dichtung eingelegt ist, und wobei beschrieben wird, dass die beiden Scheiben durch beliebige geeignete Mittel zusammengehalten werden können, zum Beispiel mit Hilfe einer Clip-Anordnung oder durch Verkleben der Scheibenränder mit einer gesonderten rohrförmigen Dichtung.

US-A-3,841,740 beschreibt ein Plasma- oder Flüssigkristall-Display mit zwei Scheiben, die durch ein Dichtmaterial voneinander auf Abstand gehalten werden, um zu vermeiden, dass Entladungsgase austreten. Die beiden Scheiben sind starr, sowohl jeweils für sich genommen als auch im Verbund.

DE 39 35 994 A offenbart ein Verfahren zum Verbinden zweier Glastafeln zu einer Isolierglasscheibe. Dabei ist vorgesehen, dass die Scheiben mittels eines äußeren Teilstrangs mit einem 2-Komponenten-Kleber verklebt werden, wobei zusätzlich noch ein innerer Teilstrang vorgesehen ist, in den ein Trockenmittel eingelagert ist.

Hiervon ausgehend liegt der Erfindung die Aufgabe zugrunde, eine Sichtscheibe der eingangs genannten Art so weiterzubilden, dass sie einerseits vorteilhafte Gebrauchseigenschaften und ein gutes optisches Erscheinungsbild und andererseits eine schnelle und kostengünstige Herstellbarkeit aufweist.

Diese Aufgabe wird erfindungsgemäß gelöst, dass die Scheiben mittels des Abstandshalte-Dichtungs-Elements, welches aus einem elastischen Silikondichtstoff auf der Basis eines Silikonkautschuk-Acetatsystems oder aus einem Dichtstoff auf Polyurethanbasis besteht, miteinander verklebt sind.

Das Abstandhalte-Dichtungs-Element wird als warmplastischer Wulst auf gebracht und dient dementsprechend gleichzeitig als Klebstoff.

Der elastische Silikondichtstoff ist vorzugsweise auf der Basis eines Silikonkautschuk-Acetat-Systems ausgebildet. Ein solches Material zeichnet sich durch eine hohe Flexibilität aus, die für die residierende Flexibilität des Scheibenverbunds von großer Bedeutung ist.

Mit Vorteil hat der Silikondichtstoff eine Dichte von ca. 1,0 g/ml und/oder eine Shore-Härte A von ca. 20 und/oder eine zulässige Gesamtverformung von ca. 25 % und/oder ein Rückstellvermögen > 98 %.

Der Dichtstoff auf Polyurethanbasis weist vorteilhafter Weise eine Dichte von ca. 1 g/cm3 und/oder eine Härte von ca. 1 shore A und/oder eine Viskosität von ca. 10.500 mPas auf.

Die innere Scheibe kann beidseitig mit einer beschlaghemmenden Beschichtung versehen sein. Bei herkömmlichen Verklebungstechniken kann nur mit einer einseitigen Beschichtung gearbeitet werden, was herstellungstechnisch erheblich aufwendiger ist. Die erfindungsgemäße Ausgestaltung führt auch bei einer beidseitigen Beschichtung zu einem stabilen Verbund.

Zur Herstellung einer erfindungsgemäßen Sichtscheibe dient ein Verfahren, bei welchem vorgesehen ist, dass auf eine der beiden die spätere Doppelscheibe bildenden Scheiben mittels einer Düse ein umlaufender Strang aus dem Material des Abstandshalte-Dichtungs-Elements aufgebracht und anschließend bei noch weichem Material die jeweils zweite Scheibe positioniert und derart angedrückt wird, dass sich nach dem Aushärten das Abstandshalte-Dichtungs-Element mit gewünschter Form und Dicke unter Verbindung der beiden Scheiben miteinander ausbildet.

Vorzugsweise wird der Strang aus dem das Abstandshalte-Dichtungs-Element ausbildende Material mittels einer CNC-gesteuerten Spritzdüse aufgebracht.

Dies kann günstigerweise so realisiert werden, dass die Herstellung der Sichtscheibe auf einen taktweise um jeweils 90° rotierenden Drehteller derart erfolgt, dass aus einem Magazin eine erste Scheibe, insbesondere die äußere Scheibe, auf dem Drehteller positioniert wird, der Drehteller um 90° in eine zweite Position gedreht wird, in dieser zweiten Position das Material des Abstandshalte-Dichtungs-Elements mittels einer CNC-gesteuerten Düse aufgebracht wird, der Teller anschließend um 90° in eine dritte Position gedreht wird, in der aus einem Magazin die zweite Scheibe, insbesondere die innere Scheibe, auf das weiche Material des Abstandshalte-Dichtungs-Elements mit definiertem Druck unter Ausbildung einer definierten Dicke des Abstandshalte-Dichtungs-Elements aufgebracht wird, der Drehteller anschließend in eine vierte Position verfahren und aus dieser vierten Position die fertige Sichtscheibe in ein Ablagemagazin übergeben wird.

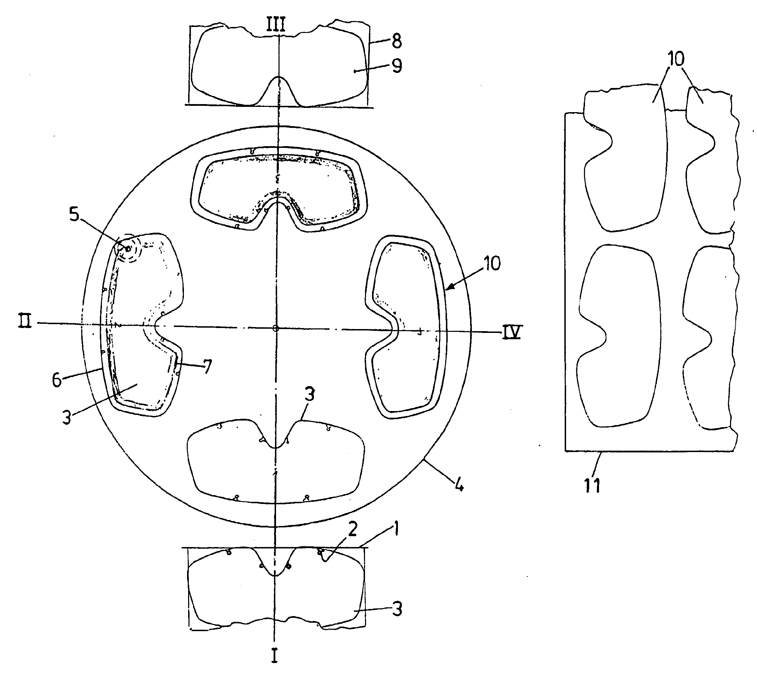

Nachfolgend wird die Erfindung anhand eines bevorzugten Ausführungsbeispiels in Verbindung mit der Zeichnung näher erläutert. Die Zeichnung zeigt eine schematische Draufsicht zur Veranschaulichung der Herstellung einer erfindungsgemäßen Brille.

Aus einem Vorratsmagazin 1 wird eine gestanzte, mit sogenannten Schlüssellöchern 2 zur späteren Befestigung am Rahmen einer Skibrille versehene Scheibe 3 entnommen, die später die äußere Scheibe bildet und auf einer ersten Position I auf einen taktweise um jeweils 90° schwenkbaren Drehteller 4 positioniert wird.

Der Drehteller wird dann in eine um 90° versetzte zweite Position II verfahren, wo mittels einer CNC-gesteuerten Spritzdüse 5 plastisches, gießfähiges Material längs des Außenrandes 6 der äußeren Sichtscheibe 3 mit Abstand zu diesem Rand unter Ausbildung eines Abstandshalte-Dichtungs-Elements 7 aufgespritzt wird.

Solange der aufgespritzte Wulst noch plastisch und verformbar ist, wird der Drehteller 4 um 90° in eine dritte Position III verdreht, wo aus einem Magazin 8 die gestanzte, später innenliegende zweite Scheibe 9 entnommen und mit definiertem Andruck auf die erste Sichtscheibe 3 aufgedrückt wird, so dass der dort vorhandene, noch plastisch verformbare Wulst unter Ausbildung zweier planparalleler Stirnseiten verformt wird. Dabei wirkt das so gebildete Abstandshalte-Dichtungs-Element 7 gleichzeitig als eine Art Klebstoff zum Verbinden von innerer Scheibe 3 und äußerer Scheibe 9, ohne das ein gesonderter bzw. zusätzlicher Klebstoffauftrag erforderlich wäre.

Anschließend wird die so gebildete fertige Sichtscheibe 10 in einer um 90° weitergedrehten vierten Position IV entnommen und in ein Ablagemagazin 11 abgelegt.

Die äußere Scheibe 3 besteht aus Polycarbonat und ist kratzfest beschichtet. Die innere Scheibe besteht aus Cellulosepropionat und ist beidseitig beschlagfrei beschichtet.

Das Abstandshalte-Dichtungs-Element besteht aus einem Einkomponenten-Dichtstoff auf der Basis von Silikonkautschuk eines Silikonkautschuk-Acetat-Systems. Im ausgehärteten Zustand besitzt dieses Material eine hohe Flexibilität.

Weiterhin weist das ausgehärtete Abstandshalte-Dichtungs-Element folgende Eigenschaften auf:
Dichte: 1,0 g/ml
Hautbildezeit: ca. 10 bis 15 Minuten
Shore-Härte A: ca. 20
Zugspannung bei 100 % Dehnung: ca. 0,4 N/mm²
Temperaturbeständigkeit: - 40°C bis + 180°C
Zulässige Gesamtverformung: 25 %
Rückstellvermögen: > 98 %

## Patentansprüche

1. Sichtscheibe für eine Skibrille umfassend eine äußere und eine innere Scheibe, wobei die äußere Scheibe (3) aus Cellulosepropionat, Celluloseacetat oder Polycarbonat und die innere Scheibe (9) aus Cellulosepropionat oder Celluloseacetat besteht, welche über ein längs des Randbereiches umlaufendes, elastisches, Abstandshalte-Dichtungs-Element miteinander verklebt sind, **dadurch gekennzeichnet, dass** die Scheiben (3, 9) mittels des Abstandshalte-Dichtungs-Elements (7), welches aus einem elastischen Silikondichtstoff auf der Basis eines Silikonkautschuk-Acetatsystems oder aus einem Dichtstoff auf Polyurethanbasis besteht, miteinander verklebt sind.

2. Sichtscheibe nach Anspruch 1, **dadurch gekennzeichnet, dass** der Silikondichtstoff eine Dichte von ca. 1,0 g/ml und/oder eine Shore-Härte A von ca. 20 und/oder eine zulässige Gesamtverformung von ca. 25 % und/oder ein Rückstellvermögen > 98 % aufweist.

3. Sichtscheibe nach Anspruch 1, **dadurch gekennzeichnet, dass** der Dichtstoff auf Polyurethanbasis eine Dichte von ca. 1 g/cm3 und/oder eine Härte von ca. 1 shore A und/oder eine Viskosität von ca. 10.500 mPas aufweist.

4. Sichtscheibe nach Anspruch 1, **dadurch gekennzeichnet, dass** die innere Scheibe(9)beidseitig mit einer beschlaghemmenden Beschichtung versehen ist.

5. Verfahren zur Herstellung einer Sichtscheibe nach einem der Ansprüche 1 bis 4, **dadurch gekennzeichnet, dass** auf eine der beiden die spätere Doppelscheibe bildenden Scheiben mittels einer Düse ein umlaufender Strang aus dem Material des Abstandshalter-Dichtungs-Elements aufgebracht und anschließend bei noch weichem Material die jeweils zweite Scheibe positioniert und derart aufgesetzt wird, dass sich nach dem Aushärten das Abstandshalte-Dichtungs-Element mit gewünschter Form und Dicke unter Verbindung der beiden Scheiben miteinander ausbildet.

6. Verfahren zur Herstellung einer Sichtscheibe nach Anspruch 5, **dadurch gekennzeichnet, dass** der Strang aus dem das Abstandshalte-Dichtungs-Element ausbildenden Material mittels einer CNC-gesteuerten Spritzdüse aufgebracht wird.

7. Verfahren zur Herstellung einer Sichtscheibe nach Anspruch 5, **dadurch gekennzeichnet, dass** die Herstellung der Sichtscheibe auf einem taktweise um jeweils 90° rotierenden Drehteller derart erfolgt, dass aus einem Magazin eine erste Scheibe, insbesondere die äußere Scheibe, auf dem Drehteller positioniert wird, der Drehteller um 90° in eine zweite Position gedreht wird, in dieser zweiten Position das Materials des Abstandshalte-Dichtungs-Elements mittels einer CNC-gesteuerten Düse aufgebracht wird, der Drehteller anschließend um 90° in eine dritte Position gedreht wird, in der aus einem Magazin die zweite Scheibe, insbesondere die innere Scheibe, auf das weiche Material des Abstandshalte-Dichtungs-Elements mit definiertem Druck unter Ausbildung einer definierten Dicke des Abstandshalte-Dichtungselements aufgebracht wird, der Drehteller anschließend eine vierte Position verfahren und aus dieser vierten Position die fertige Sichtscheibe in ein Ablagemagazin übergeben wird.

## Claims

1. Sight piece for skiing goggles comprising a distal sheet and a proximal sheet, with the distal sheet (3) consisting of cellulose propionate, cellulose acetate or polycarbonate and the proximal sheet (9) of cellulose propionate or cellulose acetate, which are united by a flexible seal and spacer element that encircles the edge, **characterized in that** the sheets (3, 9) are glued together by the seal and spacer element (7), which consists of a flexible silicone sealant on the basis of a silicone rubber acetate system or of a polyurethane based sealant.

2. Sight piece according to claim 1, **characterized in that** the silicone sealant has a density of approximately 1.0 g/ml and/or a Shore hardness A of approximately 20 and/or a permissible overall deformation of approximately 25 % and/or a retroactivity > 98 %.

3. Sight piece according to claim 1, **characterized in that** the polyurethane based sealant has a density of approximately 1.0 g/cm³ and/or a hardness of approximately 1 shore A and/or a viscosity of approximately 10.500 mPa.

4. Sight piece according to claim 1, **characterized in that** the proximal sheet (9) is provided with an anti-mist coating on both sides.

5. Method for the manufacture of a sight piece according to any one of the claims 1 to 4, **characterized in that** on one of the two sheets later forming the double sight piece, an encircling strand of the material of the seal and spacer element is applied by means of a nozzle and **in that**, with the material still soft, the second sheet is then positioned and placed on such that, after curing, the seal and spacer element of desired shape and thickness forms, uniting the two sheets.

6. Method for the manufacture of a sight piece according to claim 5, **characterized in that** the strand of the material of the seal and spacer element is applied by a CNC-controlled spray nozzle.

7. Method for the manufacture of a sight piece according to claim 5, **characterized in that** the manufacture of the sight piece takes place on a turntable which rotates intermittently by 90° at a time so that a first sheet, in particular the distal sheet, is taken from a depot and positioned on the turntable; the turntable is rotated by 90° into a second position; in this second position, the material of the seal and spacer element is applied by a CNC-controlled nozzle; the turntable is then rotated by 90° into a third position, in which the second sheet, in particular the proximal sheet, is taken from a depot and placed on the soft material of the seal and spacer element by defined pressure for a defined thickness of the seal and spacer element to form; the turntable is then moved into a fourth position from which the finished sight piece is discharged into a storing depot.

## Revendications

1. Verre pour lunettes de ski comprenant un verre extérieur et un verre intérieur, le verre extérieur (3) étant en propionate de cellulose, acétate de cellulose ou polycarbonate et le verre intérieur (9) étant en propionate de cellulose ou acétate de cellulose, qui sont collés ensemble par le biais d'un élément d'étanchéification d'écartement élastique, disposé le long de la périphérie, **caractérisé en ce que** les verres (3, 9) sont collés ensemble à l'aide d'un élément d'étanchéification d'écartement (7) qui est en un matériau d'étanchéité en silicone élastique à base d'un système acétate de caoutchouc de silicone ou en un matériau d'étanchéité à base de polyuréthane.

2. Verre selon la revendication 1, **caractérisé en ce que** le matériau d'étanchéité en silicone présente une densité d'environ 1,0 g/ml et/ou une dureté Shore A d'environ 20 et/ou une déformation totale admissible d'environ 25 % et/ou une reprise élastique > 98 %.

3. Verre selon la revendication 1, **caractérisé en ce que** le matériau d'étanchéité à base de polyuréthane présente une densité d'environ 1 g/cm3 et/ou une dureté d'environ 1 shore A et/ou une viscosité d'environ 10 500 mPas.

4. Verre selon la revendication 1, **caractérisé en ce que** le verre intérieur (9) est doté des deux côtés d'un revêtement anti-buée.

5. Procédé de fabrication d'un verre selon l'une quelconque des revendications 1 à 4, **caractérisé en ce qu'**un cordon périphérique dans le matériau de l'élément d'étanchéification d'écartement est appliqué sur un des deux verres formant ensuite le verre double à l'aide d'une buse et ensuite lorsque le matériau est encore mou respectivement le deuxième verre est positionné et placé de sorte à former ensemble après durcissement l'élément d'étanchéification d'écartement avec la forme et l'épaisseur souhaitées en reliant les deux verres.

6. Procédé de fabrication d'un verre selon la revendication 5, **caractérisé en ce que** le cordon dans le matériau formant l'élément d'étanchéification d'écartement est appliqué à l'aide d'une buse de pulvérisation à commande CNC.

7. Procédé de fabrication d'un verre selon la revendication 5, **caractérisé en ce que** le verre est fabriqué sur un plateau tournant de manière cadencée respectivement de 90°, de sorte qu'un premier verre, en particulier le verre extérieur, est positionné sur le plateau tournant à partir d'un magasin, le plateau tournant est tourné de 90° dans une deuxième position, dans cette deuxième position, le matériau de l'élément d'étanchéification d'écartement est appliqué au moyen d'une buse à commande CNC, le plateau tournant est ensuite tourné de 90° dans une troisième position, dans laquelle le deuxième verre, en particulier le verre intérieur, est appliqué à partir d'un magasin sur le matériau mou de l'élément d'étanchéification d'écartement avec une pression définie en formant une épaisseur définie de l'élément d'étanchéification d'écartement, le plateau tournant est ensuite déplacé dans une quatrième position et de cette quatrième position, le verre terminé est remis à un magasin de dépôt.
